(19) Europäisches Patentamt
European Patent Office
Office européen des brevets

(11) **EP 2 229 875 A1**

(12) **EUROPEAN PATENT APPLICATION**

(43) Date of publication:
**22.09.2010 Bulletin 2010/38**

(51) Int Cl.:
***A61B 5/00*** *(2006.01)*

(21) Application number: **09290792.2**

(22) Date of filing: **15.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **18.03.2009 KR 20090023156**

(71) Applicant: **Korea Institute of Science and Technology**
**Seongbuk-gu, Seoul 136-790 (KR)**

(72) Inventors:
- **Choi, Jee Hyun**
  **Seongbuk-gu, Seoul 136-130 (KR)**
- **Shin, Hee Sup**
  **Gyeonggi-do 437-040 (KR)**
- **Kim, Guk Bae**
  **Seongbuk-gu, Seoul 136-150 (KR)**
- **Sung, Ho-Kun**
  **Gyeonggi-do 443-470 (KR)**

(74) Representative: **Novagraaf Technologies**
**122 rue Edouard Vaillant**
**92593 Levallois-Perret Cedex (FR)**

(54) **Apparatus for detecting brain conditions**

(57)     Disclosed embodiments relate to an apparatus for detecting brain conditions. The apparatus for detecting brain conditions may include: a layer which is located adjacent to the brain of a living body; a light source which is formed on the layer and irradiates light to the brain; and an optical sensor which is formed on the layer adjacent to the light source and detects the light scattered from the brain. Since detection is possible at a location relatively adjacent to the brain, the apparatus for detecting brain conditions may improve accuracy and reliability of detection. The apparatus for detecting brain conditions may be used to detect brain conditions such as cerebral oxygenation, cerebral blood volume, cerebral blood flow, etc. or to monitor brain activities, or to diagnose and/or localize the disease foci in case of neurovascular disease such as stroke including hemorrhage or bleeding, into or around the brain or brain tumors or epilepsy, etc. Since the apparatus for detecting brain conditions has a relatively flexible structure, damage to the brain tissue may be minimized.

FIG. 6

**Description**

## CROSS-REFERENCE TO RELATED APPLICATION

This application claims priority to Korean Patent Application No. 10-2009-23156, filed on 2009.3.18, and all the benefits accruing therefrom under 35 U.S.C. §119, the contents of which in its entirety are herein incorporated by reference.

## BACKGROUND

**1. Field**

Embodiments of the disclosure relate to an apparatus for detecting brain conditions.

**2. Description of the Related Art**

In the diagnosis of cerebrovascular diseases such as epilepsy, stroke or cerebral infarction and degenerative brain diseases such as dementia or Parkinson's disease, brain activities or cerebral oxygen saturation or cerebral blood parameters (hemoglobin concentrations, blood volume, blood flow, cerebral metabolic rate of oxygen, etc) may be used as indices. By continuously monitoring the brain activities or cerebral oxygen saturation or cerebral blood parameters, acutely occurring events may be detected or prevented. As a consequence, death or brain death caused by brain diseases may be reduced or prevented.

An existing apparatus used to measure cerebral oxygen saturation does so using near infrared by mounting an optical sensor on the scalp. The apparatus irradiates light to the brain passing through the scalp and the skull, and detects the light as it is scattered from the brain and returns passing through the scalp and the skull. However, when such an apparatus is used, most of the near infrared light remains in the scalp or the skull, not passing through the brain. As a result, the intracranial apparatus as we suggest in this claim, collects the information of brain exclusively.

## SUMMARY

The embodiments of this disclosure may provide an apparatus capable of detecting brain conditions such as blood flow rate, oxygen saturation, etc. with improved accuracy and reliability as embedded at a location adjacent to the brain of humans or animals.

In an aspect, the apparatus for detecting brain conditions may include: a layer which is located adjacent to the brain; a light source which is formed on the layer and irradiates light to the brain; and an optical sensor which is formed on the layer and detects the light scattered from the brain.

The apparatus for detecting brain conditions according to the embodiments of the disclosure is capable of detecting brain conditions at a location relatively adjacent to the brain, and thus may improve accuracy and reliability of detection. The apparatus for detecting brain conditions may be used to detect brain conditions such as blood flow rate, oxygen saturation, etc., and thus may immediately cope with emergency situations. Further, it may be employed in various applications including neurosurgery, surgery, internal medicine, emergency medicine, or the like since monitoring of brain activities is possible. In addition, since it has a relatively small size and weight in addition to its flexibility, it may minimize damage to the brain tissue.

## BRIEF DESCRIPTION OF THE DRAWINGS

The above and other aspects, features and advantages of the disclosed exemplary embodiments will be more apparent from the following detailed description taken in conjunction with the accompanying drawings in which:

FIG. 1 is a schematic perspective view of an apparatus for detecting brain conditions according to an embodiment;
FIG. 2 is a graph showing the absorption spectrum of oxyhemoglobin and deoxyhemoglobin according to wavelength;
FIGS. 3a to 3c are schematic cross-sectional views of apparatuses for detecting brain conditions according to embodiments, in which a light source and an optical sensor are integrated in a layer;
FIGS. 4a to 4d schematically show the arrangement of light sources and optical sensors in apparatuses for detecting brain conditions according to embodiments;
FIG. 5 is a graph showing the waveform of a power for driving a plurality of light sources according to time in an apparatus for detecting brain conditions according to an embodiment; and
FIG. 6 is a schematic view illustrating an apparatus for detecting brain conditions according to an embodiment embedded in the human body.

## DETAILED DESCRIPTION

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like does not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In the drawings, like reference numerals in the drawings denote like elements. The shape, size and regions, and the like, of the drawing may be exaggerated for clarity.

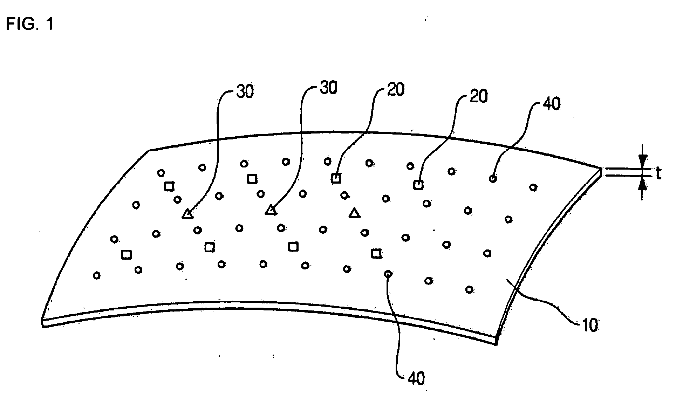

FIG. 1 is a schematic perspective view of an apparatus for detecting brain conditions according to an embodiment.

Referring to FIG. 1, an apparatus for detecting brain conditions may include a layer 10, and a light source 20 and an optical sensor 30 formed on the layer 10. The apparatus for detecting brain conditions may be embedded at a location relatively adjacent to the brain of humans or animals.

The layer 10 may be prepared to have a size and thickness appropriate for insertion into a living body by means of nanofabrication. For example, the layer 10 may have a thickness t 300 $\mu$m or smaller, preferably 30 $\mu$m or smaller. The layer 10 may be made of an organic material or an inorganic material. Further, the layer 10 may be made of a flexible material so that it may be bent depending on the brain's motion. For example, the layer 10 may be made of polyimide, polydimethylsiloxane (PDMS), or other suitable material.

A light source 20 and an optical sensor 30 may be formed on the layer 10. The light source 20 and the optical sensor 30 may be located on the surface of the layer 10 or may be embedded in the layer 10 without being exposed on the surface. The light source 20 is a device for emitting light. The light emitted from the light source 20 may be irradiated to the brain. The light source 20 may include an appropriate light-emitting element such as, for example, an organic light-emitting element or an inorganic light-emitting element. Further, the light source 20 may include a device capable of irradiating near infrared light.

In an embodiment, the light source 20 may include a device capable of irradiating a plurality of lights with different wavelengths. For example, the light source 20 may be capable of irradiating a first light with a wavelength from about 650 nm to about 800 nm and a second light with a wavelength from about 800 nm to about 900 nm at the same time.

The light emitted from the light source 20 may be irradiated into the brain passing through the surface of the brain. The light irradiated into the brain is scattered. All or part of the scattered light may return out of the brain.

As the light irradiated to the brain by the light source 20 is scattered from the brain and returns, the optical sensor 30 may detect the returning light. The optical sensor 30 may include a device capable of detecting the light from the light source 20 according to a type of the light source 20. For example, the optical sensor 30 may include a micro photodiode.

In an embodiment, the light source 20 and the optical sensor 30 may be plural, respectively. Further, a plurality of light sources 20 and optical sensors 30 may be arranged in the layer 10 in arrays. In this case, each optical sensor 30 may detect light from one or more adjacent light source(s) 20. Further, a plurality of light sources 20 may irradiate light with a time difference in a time multiplex mode.

By detecting the light emitted from the light source 20 and scattered from the brain using the optical sensor 30, brain conditions such as blood flow rate, oxygen saturation, etc. may be detected. For example, in the near infrared region, the brain tissue has a relatively low absorption rate $\mu_a$ and a relatively high scattering rate $\mu_s$. The intensity $\Psi$ of the light scattered from the brain under such a physical condition satisfies Equation 1.

## Equation 1

$$\left(\nabla^2 + k^2\right)\Psi(r,t) = -\frac{1}{D}S_o(r,t) \quad where \quad D = \left(3\left(\mu_s' + \mu_a\right)\right)^{-1},$$

$$k^2 = \frac{-\mu_a + i\omega t}{D}.$$

In Equation 1, $S_0(r, t)$ is a function of the light from the light source 20, for a spatial vector r and time t.

According to the modified Beer-Lambert law, the absorption coefficient by a medium for light propagation is proportional to the sum of products of concentration of light-absorbing molecule and the extinct coefficient of the molecule. The absorption coefficient is dependent on the wavelength of light; hence application of multiple wavelengths of light is capable of quantifying the concentrations of different types of absorbing molecule with known absorption spectrum. Since hemoglobin is the main light-absorbing species in the living body tissue in near infrared range, the absorption rate $\mu_a$ in Equation 1 is approximated by summation of absorption due to hemoglobin molecules. Since the absorption spectra of hemoglobin molecules are different depending on its oxygenation states, the absorption rate $\mu_a$ is expressed as

## Equation 2

$$\mu_a^\lambda = \sum_n \varepsilon_n^\lambda c_n \cong \varepsilon_{O_2Hb}^\lambda \left[O_2Hb\right] + \varepsilon_{HHb}^\lambda \left[HHb\right],$$

In Equation 2, by the modified Beer-Lambert law, where $\varepsilon^\lambda$ is the extinct coefficient of the corresponding molecule at wavelength $\lambda$, c is the concentration of the light absorbing molecule, and [] denotes the concentration of the molecule. That is, $[O_2Hb]$ and $[HHb]$ are the concentrations of oxyhemoglobin and deoxyhemoglobin, respectively, and $\varepsilon^\lambda_{O2Hb}$ and $\varepsilon^\lambda_{HHb}$ are the extinction coefficients of oxyhemoglobin and deoxyhemoglobin, respectively.

If the light source 20 emits two or more lights with different wavelengths, the solutions of Equation 2 can be found. With the calculated concentrations of oxyhemoglobin and deoxyhemoglobin in the brain, the oxygen saturation $S_tO_2$ of the brain can be calculated by Equation 3.

## Equation 3

$$S_tO_2 = \frac{\left[O_2Hb\right]}{\left[O_2Hb\right] + \left[HHb\right]} \times 100(\%).$$

The wavelengths of the lights irradiated by the light source 20 may be determined based on the absorption spectrum of hemoglobin. For example, by employing two or more wavelengths at which the difference in absorption rates of oxyhemoglobin and deoxyhemoglobin is relatively large, the concentrations of oxyhemoglobin and deoxyhemoglobin may be obtained with a relatively smaller error.

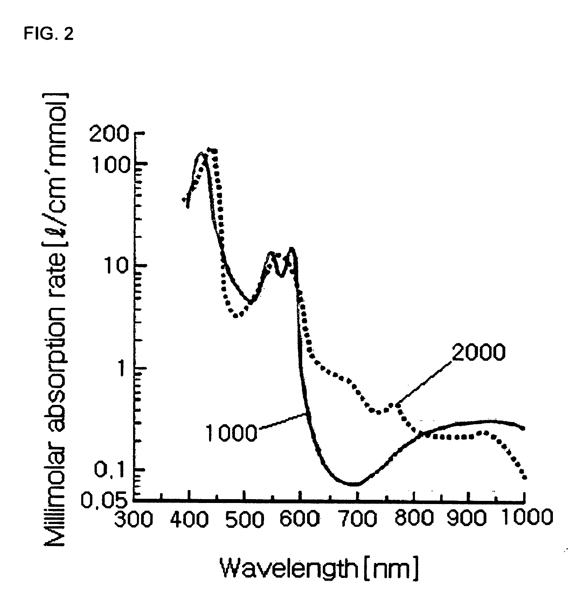

FIG. 2 is a graph showing the absorption spectrum of oxyhemoglobin and deoxyhemoglobin according to wavelength in the visible and near infrared regions. The curve 1000 shows the absorption spectrum of oxyhemoglobin, and the curve 2000 shows the absorption spectrum of deoxyhemoglobin.

Referring to FIG. 2, in the wavelength region from about 650 nm to about 800 nm, deoxyhemoglobin has a relatively larger absorption rate than oxyhemoglobin. On the contrary, in the wavelength region from about 800 nm to about 900 nm, oxyhemoglobin has a relatively larger absorption rate than deoxyhemoglobin. Accordingly, in an embodiment, the light source 20 may irradiate a first light with a wavelength from about 650 nm to about 800 nm and a second light with a wavelength from about 800 nm to about 900 nm at the same time, so that the concentrations of oxyhemoglobin and deoxyhemoglobin may be determined with a relatively small error.

As described above, the concentrations of oxyhemoglobin and deoxyhemoglobin may be determined based on the light detected by the optical sensor 30, and the oxygen saturation of the brain may be determined based on the concentrations of oxyhemoglobin and deoxyhemoglobin. The sum of the concentrations of oxyhemoglobin and deoxyhemoglobin is the total hemoglobin concentration, and is proportional to the blood flow rate. Accordingly, the apparatus for detecting brain conditions according to an embodiment may be used to detect brain conditions, including the blood flow rate.

The light source 20 and the optical sensor 30 may be integrated into the layer 10 by means of nanofabrication. In this regard, the light source 20 and the optical sensor 30 may have a nanometer scale size, so that the weight of the apparatus for detecting brain conditions is 10 mg or less.

Since the light source 20 and the optical sensor 30 are integrated into the layer 10 by means of nanofabrication, the apparatus for detecting brain conditions may be manufactured into a size appropriate for insertion into a living body. Because the apparatus for detecting brain conditions may be embedded into a location relatively adjacent to the brain, the accuracy and reliability of detection may be improved. Furthermore, the effect of the apparatus for detecting brain conditions on the living body tissue may be minimized, and unwanted movement of the devices of the apparatus caused by the movement of the brain may be minimized.

In an embodiment, an electrode 40 may be formed in the layer 10. The electrode 40 may be in contact with the brain and may serve to detect electrical signals from brain cells. Like the light source 20 and the optical sensor 30, the electrode 40 may also be plural. Further, a plurality of electrodes 40 may be arranged in the layer 10 in arrays. By analyzing the electrical signals detected by a plurality of electrodes 40, brain activities may be monitored by means of electrocorticography (ECoG).

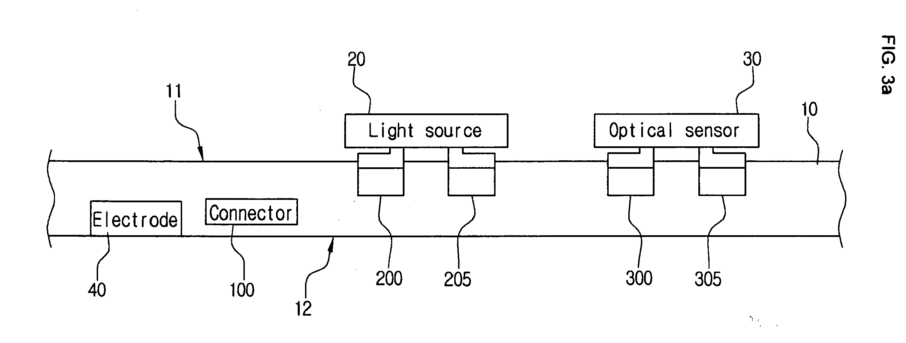

FIGS. 3a to 3c are schematic cross-sectional views of apparatuses for detecting brain conditions according to embodiments, in which a light source and an optical sensor are integrated in a layer.

FIGS. 3a to 3c are figures for explaining the location of light sources 20, 21, 22, optical sensors 30, 31, 32, an electrode 40 and a connector 100 in a layer 10. The order of arrangement of the light sources 20, 21, 22, the optical sensors 30, 31, 32, the electrode 40 and the connector 100 and the shapes thereof depicted in FIGS. 3a to 3c are exemplary. It will be understood to those skilled in the art that the actual order of arrangement or shapes of the light source, the optical sensor, the electrode and the connector may be different from the figures.

Referring to FIG. 3a, the light sources 20, the optical sensors 30, the electrodes 40 and connector 100 for driving them may be formed in a layer 10. The connector 100 serves for supplying power to control the light source 20, receiving optical and electrical signals detected by the optical sensor 30 and the electrode 40, and transmitting them to outside.

In an embodiment, the light source 20 and the optical sensor 30 may be located on an upper surface 11 of the layer 10, and the electrode 40 may be located to be exposed on a lower surface 12 of the layer 10. For example, when the apparatus for detecting brain conditions is embedded beneath the skull, the upper surface 11 of the layer 10 may be adjacent to the skull, and the lower surface 12 may be adjacent to the brain. The light source 20 and the optical sensor 30 located on the upper surface 11 of the layer 10 may irradiate light to the brain through the layer 10 or detect the light scattered from the brain. The electrode 40 may be located on the lower surface 12 of the layer 10 so that it may be in contact with the brain and detect electrical signals from the brain.

The light source 20 may include input and output terminals 200, 205 for transmitting electrical signals. Each terminal 200, 205 may be formed from one or more conductive material(s). The shape of the terminals 200, 205 depicted in FIG. 3a is exemplary, and the actual shape of the terminals may be different from the figure. Like the light source 20, the optical sensor 30 may also include input and output terminals 300, 305.

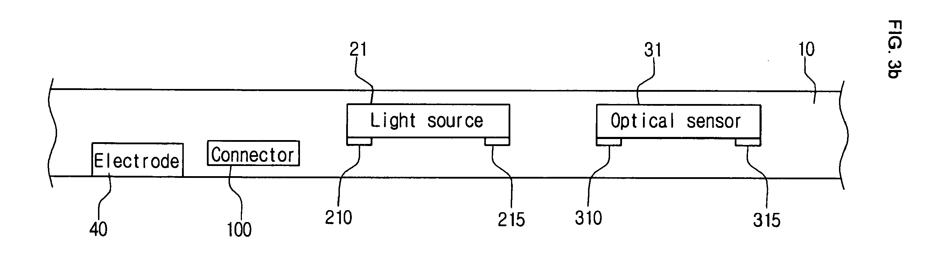

Referring to FIG. 3b, in another embodiment, a light source 21, an optical sensor 31, an electrode 40 and a connector 100 may be formed in a layer 10. In the embodiment of FIG. 3b, the light source 21 and the optical sensor 31 may be completely buried in the layer 10, without being exposed on the surface of the layer 10.

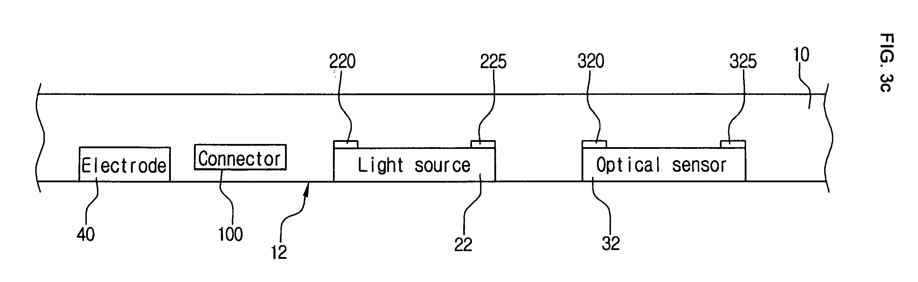

Referring to FIG. 3c, in another embodiment, a light source 22, an optical sensor 32, an electrode 40 and a connector 100 may be formed in a layer 10. In the embodiment of FIG. 3c, the light source 22 and the optical sensor 32 may be located to be exposed on a lower surface 12 of the layer 10. For example, when the apparatus for detecting brain conditions is embedded beneath the skull, the light source 22 exposed on the lower surface 12 of the layer 10 may directly irradiate light to the brain, and the optical sensor 32 may detect the light scattered from the brain directly.

In the embodiments of FIG. 3b and FIG. 3c, other configurations except for the relative location of the light source 21, 22 and the optical sensor 31, 32 are the same as the embodiment of FIG. 3a. Hence, a detailed description thereabout will be omitted.

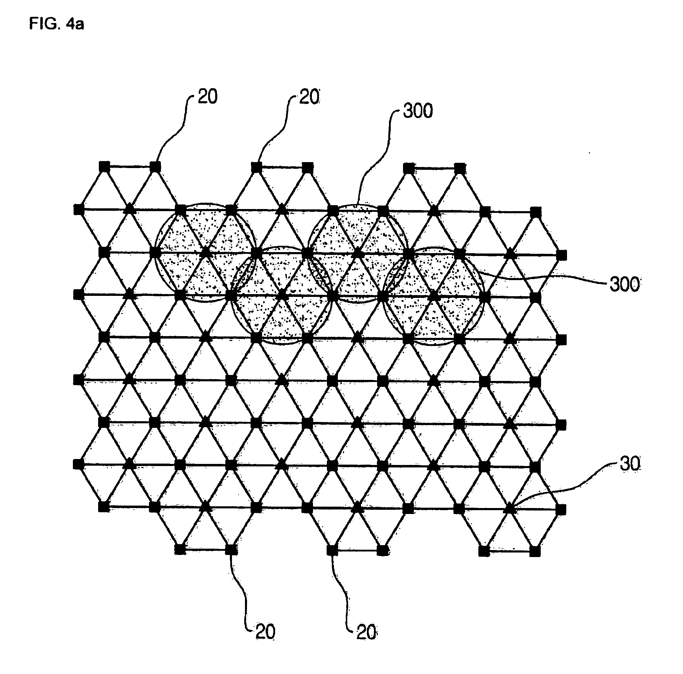

FIGS. 4a to 4d schematically show the arrangement of light sources 20 and optical sensors 30 in apparatuses for detecting brain conditions according to embodiments. FIG. 4a to 4d schematically show a portion of the surface of a layer in which light sources 20 and optical sensors 30 are formed.

Referring to FIG. 4a, light sources 20 and optical sensors 30 may be arranged to form regular hexagons on the layer surface. Six light sources 20 may be arranged to be located on each vertex of a regular hexagon, and an optical sensor 30 may be arranged to be located at the center of the regular hexagon formed by the six light sources 20. In such an arrangement, the ratio of the number of light sources 20 to that of optical sensors 30 is 2:1. The region 300 represents the detection range of the optical sensor 30 located at the center of the region 300. As seen in the figure, lights from six neighboring light sources 20 may be detected using one optical sensor 30.

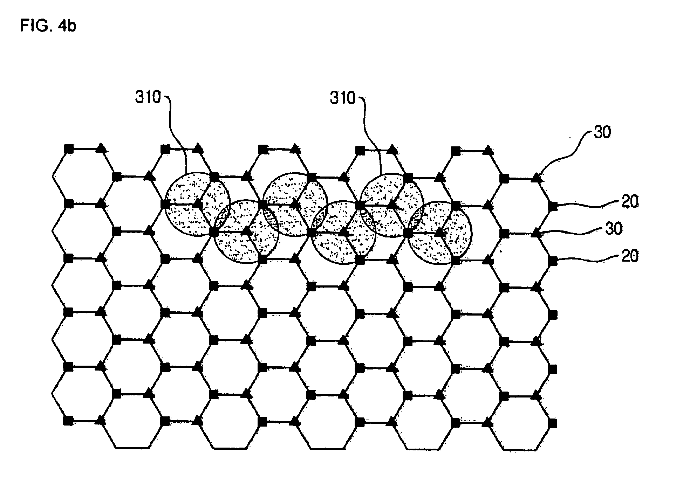

Referring to FIG. 4b, in another embodiment, light sources 20 and optical sensors 30 may be arranged to form regular hexagons in a different manner. Three light sources 20 and three optical sensors 30 may be alternately arranged to be located on each vertex of a regular hexagon. In such an arrangement, the ratio of the number of light sources 20 to that of optical sensors 30 is 1:1. The region 310 represents the detection range of the optical sensor 30 located at the center of the region 310. As seen in the figure, lights from three neighboring light sources 20 may be detected using one optical sensor 30.

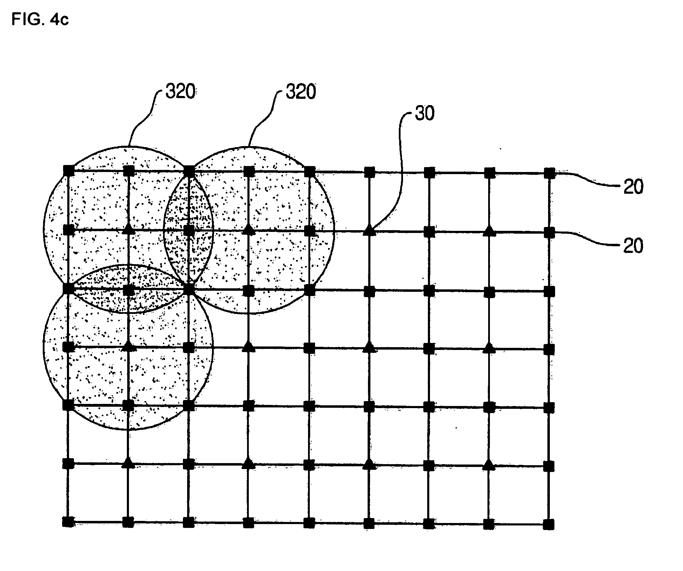

Referring to FIG. 4c, light sources 20 and optical sensors 30 may be arranged to form squares. Four light sources 20 may be arranged to be located on each vertex of a square, and four light sources 20 may be arranged at each center of four sides of the square. An optical sensor 30 may be arranged to be located at the center of the square formed by the eight light sources 20. In such an arrangement, the ratio of the number of light sources 20 to that of optical sensors 30 is 3:1. The region 320 represents the detection range of the optical sensor 30 located at the center of the region 320. As seen in the figure, lights from eight neighboring light sources 20 may be detected using one optical sensor 30.

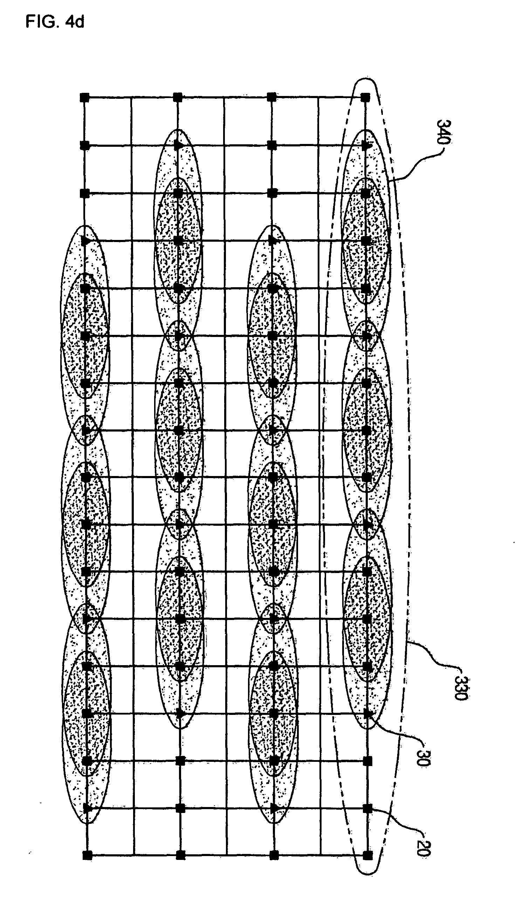

Referring to FIG. 4d, light sources 20 and optical sensors 30 may be alternately arranged in each row 330. For example, in each row 330, three light sources 20 and one optical sensor 30 may be alternately arranged. The region 340 represents the detection range of the optical sensor 30 included in the region 340. As seen in the figure, lights from six light sources 20 on both sides in the same row may be detected using one optical sensor 30.

The arrangements of light sources 20 and optical sensors 30 described referring to FIGS. 4a to 4d are examples of many possible arrangements of the light sources 20 and optical sensors 30. Without being limited thereto, a plurality of light sources 20 and optical sensors 30 may be arranged in an apparatus for detecting brain conditions in different forms.

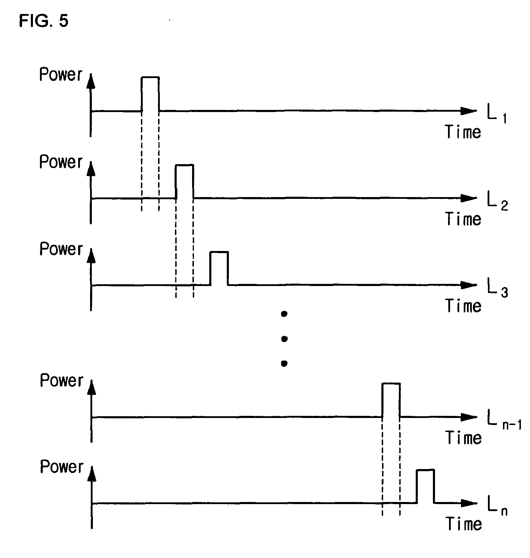

FIG. 5 is a graph showing the waveform of a power for driving a plurality of light sources in an apparatus for detecting brain conditions according to an embodiment.

An apparatus for detecting brain conditions according to an embodiment may include a plurality of light sources. The plurality of light sources may be grouped into n groups. Each group may include at least one light source, and each group may include the same or different number of light source(s). The n light source groups may operate in a time multiplex mode.

FIG. 5 shows n drive waveforms $L_1$, $L_2$, ..., $L_n$ for driving the n light source groups. When the power waveform $L_1$, $L_2$,..., $L_n$ has a high level, lights may be emitted from the light sources of the corresponding groups. When the pulse has a low level, lights may not be emitted from the light sources of the corresponding groups.

The power waveforms $L_1$, $L_2$, ..., $L_n$ may be designed to have a high level at different time, with a time difference. As a result, the n light source groups may emit light at different times. Since each light source emits light at different time, by controlling the operation time of the optical sensor corresponding to specific light sources depending on the light emission time of the light sources, it may be possible to allow each optical sensor to detect lights from specific adjacent light sources only.

The drive waveform of FIG. 5 is exemplary. The waveform of a power for driving light sources is not limited thereto, and the light sources may be driven by a power with a different waveform.

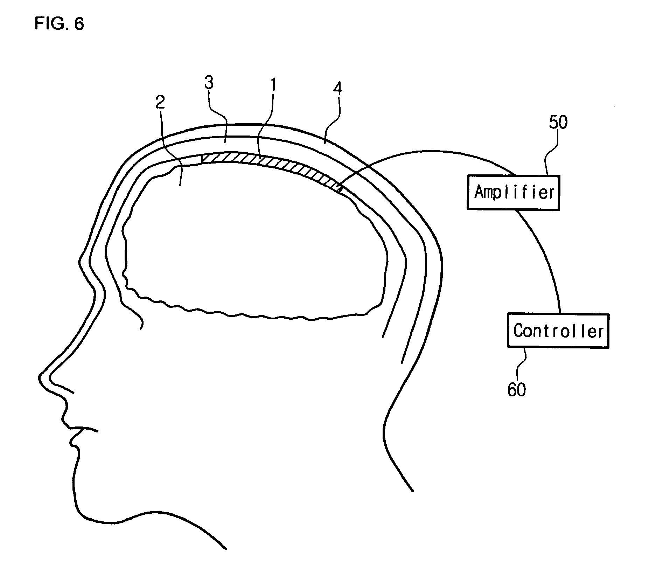

FIG. 6 is a schematic view illustrating an apparatus for detecting brain conditions according to an embodiment embedded in the human body.

Referring to FIG. 6, an apparatus 1 for detecting brain conditions may be embedded between the brain 2 and the skull 3 of a person. For example, the apparatus 1 for detecting brain conditions may be located on the surface of the cerebral cortex or on the dura or fixed to the intracranial surface of the skull. Since the apparatus 1 for detecting brain conditions is located beneath the skull 3, light may be directly irradiated to the brain 2, and scattered light and electrical signals may be detected directly from the brain 2.

The apparatus 1 for detecting brain conditions may be optically and/or electrically connected to an amplifier 50 and a controller 60 outside the human body. The amplifier 50 may amplify the light and electrical signals detected by the apparatus 1 for detecting brain conditions. By amplifying the magnitude of the light and electrical signals using the amplifier 50, detection of brain conditions may become easier.

The controller 60 may receive the light and electrical signals amplified by the amplifier 50. Using the received light or electrical signals, the controller 60 may detect brain conditions such as blood flow rate, oxygen saturation, or the like. The change in the intensity of the received light is proportional to the change in the light absorption coefficient by the brain. The change in oxygen saturation or blood flow rate may be determined using Equations 1 to 3.

In FIG. 6, the apparatus 1 for detecting brain conditions is embedded between the brain 2 and the skull 3. But, this is only an example, and the apparatus 1 for detecting brain conditions may be embedded between the skull 3 and the scalp 4. Alternatively, a portion of the skull 3 may be made thinner and the apparatus 1 for detecting brain conditions may be embedded to be located there.

While the exemplary embodiments have been shown and described, it will be understood by those skilled in the art that various changes in form and details may be made thereto without departing from the spirit and scope of this disclosure as defined by the appended claims.

In addition, many modifications can be made to adapt a particular situation or material to the teachings of this disclosure without departing from the essential scope thereof. Therefore, it is intended that this disclosure not be limited to the particular exemplary embodiments disclosed as the best mode contemplated for carrying out this disclosure, but that this disclosure will include all embodiments falling within the scope of the appended claims.

**Claims**

1. An apparatus for detecting brain conditions comprising:

   a layer which is located adjacent to the brain of a living body;
   a light source which is formed on the layer and irradiates light to the brain; and
   an optical sensor which is formed on the layer adjacent to the light source and detects the light emitted from the light scattered from the brain.

2. The apparatus for detecting brain conditions according to claim 1, wherein the light source comprises a plurality of light sources arranged in arrays.

3. The apparatus for detecting brain conditions according to claim 2, wherein the plurality of light sources emit light with time difference.

4. The apparatus for detecting brain conditions according to claim 2, wherein the optical sensor comprises a plurality of optical sensors arranged in arrays.

5. The apparatus for detecting brain conditions according to claim 4, wherein the plurality of optical sensors are located between the pluralities of light sources.

6. The apparatus for detecting brain conditions according to claim 4, wherein each of the optical sensors detects light from one or more adjacent light source(s) among the plurality of light sources.

7. The apparatus for detecting brain conditions according to claim 1, wherein the light source emits a plurality of lights with different wavelengths.

8. The apparatus for detecting brain conditions according to claim 1, wherein the layer has a thickness $300\,\mu\text{m}$ or smaller.

9. The apparatus for detecting brain conditions according to claim 1, wherein the light source and the optical sensor are embedded in the layer.

10. The apparatus for detecting brain conditions according to claim 1, wherein the light source and the optical sensor are located on the surface of the layer.

11. The apparatus for detecting brain conditions according to claim 1, further comprising an electrode which is located on the surface of the layer adjacent to the brain and detects electrical signals from the brain.

12. The apparatus for detecting brain conditions according to claim 11, wherein the electrode comprises a plurality of electrodes arranged in arrays.

FIG. 1

FIG. 2

FIG. 3a

EP 2 229 875 A1

FIG. 3b

| | | 21 | | 31 | 10 |

Electrode | Connector | Light source | Optical sensor

40 | 100 | 210 | 215 | 310 | 315

**FIG. 3c**

FIG. 4a

FIG. 4b

FIG. 4c

FIG. 4d

FIG. 5

FIG. 6

**EUROPEAN SEARCH REPORT**

Application Number

EP 09 29 0792

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/137851 A1 (CORNELL RES FOUNDATION INC [US]; UNIV GEORGETOWN [US]; MA HONGTAO [US]) 13 November 2008 (2008-11-13) * paragraphs [0002], [0003], [0011], [0017], [0023] - [0035]; figures 1,2 * ----- | 1-12 | INV. A61B5/00 |
| X | EP 0 413 588 A2 (CRITIKON INC [US]) 20 February 1991 (1991-02-20) * column 1, lines 29-45 * * column 3, lines 13-52 * * column 4, lines 38-50 * * figures 1,6a-6c * ----- | 1-5,9-11 | |
| X | WO 2008/052070 A2 (UNIV DENVER [US]; SHOURESHI RAHMAT A [US]; AASTED CHRISTOPHER [US]) 2 May 2008 (2008-05-02) * paragraphs [0007] - [0009], [0032] - [0037], [0048] - [0050], [0055], [0056], [0079]; figures 2a,3a-3c * ----- | 1-7,9,10 | |

TECHNICAL FIELDS SEARCHED (IPC)

A61B

The present search report has been drawn up for all claims

| Place of search | Date of completion of the search | Examiner |
|---|---|---|
| Berlin | 28 May 2010 | Pohjamo, Terhi |

**ANNEX TO THE EUROPEAN SEARCH REPORT
ON EUROPEAN PATENT APPLICATION NO.**

EP 09 29 0792

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2010

| Patent document cited in search report | | Publication date | Patent family member(s) | | Publication date |
|---|---|---|---|---|---|
| WO 2008137851 | A1 | 13-11-2008 | NONE | | |
| EP 0413588 | A2 | 20-02-1991 | AT | 134849 T | 15-03-1996 |
| | | | AT | 148323 T | 15-02-1997 |
| | | | CA | 2023355 A1 | 18-02-1991 |
| | | | DE | 69025673 D1 | 11-04-1996 |
| | | | DE | 69025673 T2 | 01-08-1996 |
| | | | DE | 69029869 D1 | 13-03-1997 |
| | | | DE | 69029869 T2 | 05-06-1997 |
| | | | DK | 0413588 T3 | 22-07-1996 |
| | | | DK | 0651968 T3 | 24-02-1997 |
| | | | EP | 0651968 A1 | 10-05-1995 |
| | | | ES | 2084662 T3 | 16-05-1996 |
| | | | ES | 2097063 T3 | 16-03-1997 |
| | | | GR | 90100617 A | 30-12-1991 |
| | | | JP | 3228746 A | 09-10-1991 |
| | | | US | 5024226 A | 18-06-1991 |
| | | | US | 5127407 A | 07-07-1992 |
| WO 2008052070 | A2 | 02-05-2008 | NONE | | |

EPO FORM P0459

For more details about this annex : see Official Journal of the European Patent Office, No. 12/82

**REFERENCES CITED IN THE DESCRIPTION**

**Patent documents cited in the description**

- KR 10200923156